(19)

Europäisches
Patentamt

European
Patent Office

Office européen
des brevets

(11)     EP 4 301 812 B1

(12)                    EUROPEAN PATENT SPECIFICATION

(45) Date of publication and mention
    of the grant of the patent:
    09.04.2025  Bulletin 2025/15

(51) International Patent Classification (IPC):
    **C08L 1/02** (2006.01)          **C08K 3/22** (2006.01)
    **C08K 3/105** (2018.01)         **C08K 3/11** (2018.01)

(21) Application number: 22707770.8

(22) Date of filing: 25.02.2022

(52) Cooperative Patent Classification (CPC):
    **C08L 1/02; C08K 3/105; C08K 3/11; C08K 3/22**

(86) International application number:
    PCT/EP2022/054891

(87) International publication number:
    WO 2022/184600 (09.09.2022 Gazette 2022/36)

(54) **A METHOD FOR THE PREPARATION OF A HYBRID NANO-STRUCTURED COMPOSITE COMPRISING CELLULOSE NANO-PARTICLES AND METAL COMPOUND NANO-PARTICLES**

VERFAHREN ZUR HERSTELLUNG EINES HYBRIDEN NANOSTRUKTURIERTEN VERBUNDSTOFFS MIT CELLULOSENANOPARTIKELN UND METALLVERBUNDNANOPARTIKELN

PROCÉDÉ DE PRÉPARATION D'UN COMPOSITE HYBRIDE NANO-STRUCTURÉ COMPRENANT DES NANO-PARTICULES DE CELLULOSE ET DES NANO-PARTICULES DE COMPOSÉS MÉTALLIQUES

(84) Designated Contracting States:
    AL AT BE BG CH CY CZ DE DK EE ES FI FR GB
    GR HR HU IE IS IT LI LT LU LV MC MK MT NL NO
    PL PT RO RS SE SI SK SM TR

(30) Priority: 02.03.2021 EP 21160301

(43) Date of publication of application:
    10.01.2024  Bulletin 2024/02

(73) Proprietor: Cellicon B.V.
    6827 BX Arnhem (NL)

(72) Inventors:
  • O'CONNOR, Paul
    3871 KM Hoevelaken (NL)
  • BABICH, Igor
    7545 GM Enschede (NL)
  • O'CONNOR, Kimberley
    5254 JV Haarsteg (NL)
  • BOUTE, Bjorn
    5254 JV Haarsteg (NL)

(74) Representative: Hoyng Rokh Monegier B.V.
    Rembrandt Tower, 30th Floor
    Amstelplein 1
    1096 HA Amsterdam (NL)

(56) References cited:
  • RUSZALA M. J. A. ET AL: "Low Carbon Footprint TiO2 Substitutes in Paint: A Review", INTERNATIONAL JOURNAL OF CHEMICAL ENGINEERING AND APPLICATIONS, vol. 6, no. 5, 1 October 2015 (2015-10-01), SG, pages 331 - 340, XP055832412, ISSN: 2010-0221, Retrieved from the Internet <URL:http://dx.doi.org/10.7763/IJCEA.2015.V6.505> DOI: 10.7763/IJCEA.2015.V6.505
  • MA JINXIA ET AL: "Preparation of ZnO-cellulose nanocomposites by different cellulose solution systems with a colloid mill", CELLULOSE, SPRINGER NETHERLANDS, NETHERLANDS, vol. 23, no. 6, 8 October 2016 (2016-10-08), pages 3703 - 3715, XP036104397, ISSN: 0969-0239, [retrieved on 20161008], DOI: 10.1007/S10570-016-1081-0

- **FAROOQ AMJAD ET AL: "Cellulose from sources to nanocellulose and an overview of synthesis and properties of nanocellulose/zinc oxide nanocomposite materials", vol. 154, 1 July 2020 (2020-07-01), NL, pages 1050 - 1073, XP055832401, ISSN: 0141-8130, Retrieved from the Internet <URL:https://www.sciencedirect. com/science/article/pii/S0141813020303585/ pdfft? md5=9de9f25f8a696da56bd5ec986d73b013& pid=1-s2.0-S0141813020303585-main.pdf> DOI: 10.1016/j.ijbiomac.2020.03.163**

## Description

BACKGROUND OF THE INVENTION

### 1. Field of the Invention

This invention relates to a method for the preparation of a hybrid nano-structured composite comprising cellulose nano-particles and metal compound nano-particles, to the nano structured-composite product obtainable by the process and uses thereof.

### 2. Description of the Related Art

The production of cellulose nano-particles is known in the art. For example, Delgado-Aguilar et al in BioResources 10(3), 5345-5355 (2015) describe several processes for preparation of cellulose nanofibers from cellulose containing feedstock involving a combination of chemical pretreatment with high energy mechanical treatment. These processes use costly and non-environmentally chemicals and/or solvents, high energy consumption and complexity in several processing steps.

A known simple route to produce cellulose nano-particles uses acidic solvents ($H_2SO_4$, HCl and or $SO_2$ in water or in organic solvents). However, the yield and the quality of the obtained cellulose nano-particles product is poor because of degradation of the cellulose into sugars and other compounds which will also reduce the applicability of the obtained product.

In WO2017/055407 it is described to dissolve cellulose in a substantially proton-free inorganic molten salt solvent medium, preferably a $ZnCl_2$ hydrate, and precipitating the cellulose with an antisolvent to high aspect ratio nano-cellulose fibrils. However, the properties of the obtained cellulose nanocrystals are not as good as desired in terms of purity, crystallinity, chemical stability and mechanical properties of the product.

In WO2020212616 a further improved process is described for the preparation of micro- or nano crystalline cellulose from virgin cellulose comprising the contacting with a first solvent comprising 40 to 65 wt.% $ZnCl_2$ in water whereby the amorphous cellulose phase is preferentially dissolved over the crystalline cellulose phase producing cellulose micro-particles having an XRD type I structure in a high yield and with high crystallinity and then preferably contacting the obtained product with a second solvent comprising a higher concentration than the first solvent of between 65 and 90 wt.% $ZnCl_2$ in water to produce cellulose nano-particles having an XRD type II structure in a high yield, high crystallinity and high purity.

The production of nano-particles of a metal compound is also well known. However, the problem is to form a nano-structured composite wherein both the nano-particles of the metal compound and cellulose are mixed homogeneously on nano-scale. Nanoparticles typically have a size from about 1 to below 100 nm, preferably below 60 nm or 40 nm and most preferably even below 20 nm. For cellulose nanocrystals having a large aspect ratio this is the size range for the smaller thickness dimension, not the length dimension. A nano-structured composite is a composite where the nano-particles are homogeneously dispersed on nano-scale so most cellulose nano-particles neighbor metal compound nano-particles within the abovementioned nano-scale dimensions, which can be established by Scanning electron microscopy (SEM).

The direct mixing of the metal nano-particles with the nano-cellulose particles is not a complex process but may also cause damage and/or degradation of the cellulose nanoparticles, costs a lot of mechanical energy, but mostly the problem is that the products are not homogeneously mixed on nanoscale.

WO2019229030A1 describes a process for the preparation of hybrid inorganic-organic materials, but this process does not result in a nano-structured composite material comprising nano-particles of metal compound and cellulose. This process comprises forming a dissolution of cellulose in an ionic liquid solvent and dispersing an inorganic material, for example alumina or silica, in the cellulose solution adding an anti-solvent to precipitate the nano-cellulose together with the dispersed inorganic material. The resulting co-precipitated inorganic particles are, as opposed to the inorganic particles of the invention, not nano-particles and the hybrid composite material is not a nano-structured composite. The alumina ($Al_2O_3$), dispersed in $ZnCl_2$ together with NC-$ZnCl_2$, results in alumina particles having an average size of 100 nm or more which are not nano-particles.

Farooq e.a. in the International Journal of Biological Macromolecules 154, (2020) 1050-1073, describes hybrid composites comprising cellulose nano-particles modified with metal nano-particles, in particular Zinc-oxide nanoparticles. Farooq describes in Chapter 2 of the review article several preparation processes to produce cellulose nano-particles involving alkaline-acid treatment of cellulose biomass or enzymatic pretreatment or pretreatment with ionic liquids followed by hydrolysis and high-pressure homogenization or mechanical treatment to produce cellulosic nano-particles. Farooq also describes in Chapter 3 of the review article various processes for the preparation of metallic nano-particles wherein different morphologies are obtained in different known ways. It is described to produce Zinc-oxide nano-particles for example by a non-hydrolytic (solution) process using zinc acetate dehydrate. Farooq then also describes in Chapter 3.3 a variety of different ways to prepare the hybrid composite material including simple mixing of the cellulose nano-particles in aqueous suspension with metal oxide nano-particles without using any external reducing agent, mixing cellulose nano-particles with metal nano-particles together with reducing agent or surface modifying of the cellulose nano-particles and adding metal-oxide nanoparticles. The preparation processes all start from an aqueous suspension of cellulose nano-particles.

Ma et al. in Cellulose (2016) 23: 3703-3715 describe a process for the preparation of ZnO-cellulose nanocomposites comprising mixing a cellulose - Zinc-chloride ($ZnCl_2$) aqueous solutions with a cellulose NaOH/urea aqueous solution in a colloid mill at room temperature. The cellulose is completely dissolved in 65wt% $ZnCl_2$ solution at 80°C and also the cellulose in the NaOH/urea aqueous solution is completely dissolved. On mixing of the solutions aggregated large ZnO grains are formed by reaction of $ZnCl_2$ with hydroxide in the presence of the dissolved cellulose in amount between 0.5 wt% and 2 wt%. The presence of cellulose during ZnO precipitation is to prevent growth and agglomeration of ZnO particles. The reaction mixture is milled in a colloid mill to nano-size grains and the generated ZnO-cellulose nanocomposite was calcined at 575 °C to produce the Nano-ZnO particles. XRD measurement showed no cellulose crystallinity peaks, which indicates that in the resulting product after calcination no nano-cellulose crystals are present. A disadvantage of the process is that it involves high energy milling step, which is more complicated and costly but, more importantly, would result in degradation and discoloration of any cellulose that would be present. The resulting nanocomposites is referred to as ZnO-cellulose but only comprise ZnO nanoparticles and but no nano-cellulose crystals. The product has yellow-orange emission bands. The resulting nano-particles are not suitable as white pigment replacement.

Ruszala et al., IJCEA, vol. 6, no. 5, October 2015, 331-340 describes various alternative materials for replacing $TiO_2$ as a white pigment in paints and describes in part V.A.1 also ZnO but not ZnO nanoparticles. It is described that lack of stability and low refractive index results in ZnO is not used much in paints. Ruszala does not describe cellulose nanoparticles nor cellulose - ZnO hybrid nanoparticles.

A disadvantage of the described processes for the production of the hybrid composite is that they are complex, involving several process steps and consuming expensive chemicals and therefore are economically and environmentally less attractive.

It is therefore an object of the invention to provide a process for the preparation of a hybrid composite comprising metal nano-particles and cellulose nano-particles that is less complicated and results in a very homogeneous hybrid nano-structured composite material wherein metal nano-particles and cellulose nano-particles are homogeneously mixed on nanoscale. The process of the invention preferably has at least one of the advantages of fewer process steps, less expensive chemicals, lower processing cost, lower energy consumption and lower environmental impact.

BRIEF SUMMARY OF THE INVENTION

According to the invention at least one of the mentioned disadvantages have been overcome by providing process for preparing a hybrid nano-structured composite material comprising cellulose nanocrystalline particles and metal compound nano-particles comprising the steps of:

a) Contacting virgin cellulose with a molten metal salt solvent $M_1$-S comprising metal ions $M_1$ and dissoluting the virgin cellulose,
b) Adding a precipitation reactant B to convert at least part of the metal ions $M_1$ of the molten metal salt solvent $M_1$-S to metal compound nano-particles $M_{1-}$X or exchanging at least part of metal ions $M_1$ with metal ions $M_2$ by contacting with a solution of a salt comprising metal ions $M_2$ and precipitating metal compound $M_{2-}$X nano-particles directly or by adding a precipitation reactant B,
c) Adding an anti-solvent and precipitating the cellulose nano-particles before, during or after step b),
d) Isolating the co-precipitated cellulose- and metal compound nano-particles to obtain the hybrid nano-structured composite material,
e) Optionally converting the metal compound in the hybrid nano-structured composite material into another metal compound.

In another aspect the invention relates to the hybrid nano-structured composite material comprising metal nano-particles and cellulose nanocrystalline particles obtainable by the process of the invention having improved homogeneity and improved properties in use.

Yet another aspect of the invention relates to the use of the hybrid nano-structured composite material in food packaging, biopharmaceuticals, biomedical, cosmetics and electronics applications, for example in solar cells, flexible displays, ultracapacitors etc. where nanoparticles of metal compounds comprising metals like Li, Mn, Ti, Zn, Ru and others are combined with nanocellulose paper, foils, sheets, tapes etc.

In yet another aspect of the invention relates to the use metal nano-particles or the use of cellulose nanoparticles or most preferably the use of the hybrid nano-structured composite material of the invention as a white base pigment, for example but not limited to paint, coatings, adhesives, sealants, inks, plastics, but also in cosmetics such as toothpaste or in food products. The mentioned use relates in particular to replace Titanium compounds such as Titanium dioxide ($TiO_2$) as white pigment in the mentioned applications. It is known that nanocellulose is white and also that ZnO itself is white, but it was surprisingly found that the hybrid nano-structured composite material of the invention is even whiter than the constituting nanoparticles themselves, probably because ZnO is so well dispersed in the nano-cellulose.

BRIEF DESCRIPTION OF THE DRAWINGS

The features and advantages of the invention will be appreciated upon reference to the following drawings, in which:

FIG. 1 Example A is a schematic view of one embodiment of the process of the invention showing general process steps in the production of hybrid composite of cellulose nanocrystals and metal compound nano-particles $NC_s$ - $M_1OH$ .

FIG. 2 Example A1 is a specific embodiment of the process of example A showing the use of $ZnCl_2.4H_2O$ as the molten salt solvent to produce $NC_s$ -$Zn(OH)_2$ hybrid composite.

FIG. 3 Example B is a general schematic view of another embodiment of the process of the invention to produce a $NC_s$ - $M_1HCO_3$ hybrid composite.

FIG. 4 Example B1 is a specific embodiment of the process Example B to produce $NC_s$ - $Zn(HCO_3)_2$ hybrid composite.

FIG. 5 Example C is a general schematic view of another embodiment of the process of the invention to produce $NC_s$ - $M_2$ -Acetate hybrid composite.

FIG. 6 Example C1 is a specific schematic view of one embodiment of the process of the invention of example C to produce $NC_s$ - Li-Acetate hybrid composite.

DETAILED DESCRIPTION OF CERTAIN EMBODIMENTS OF THE INVENTION

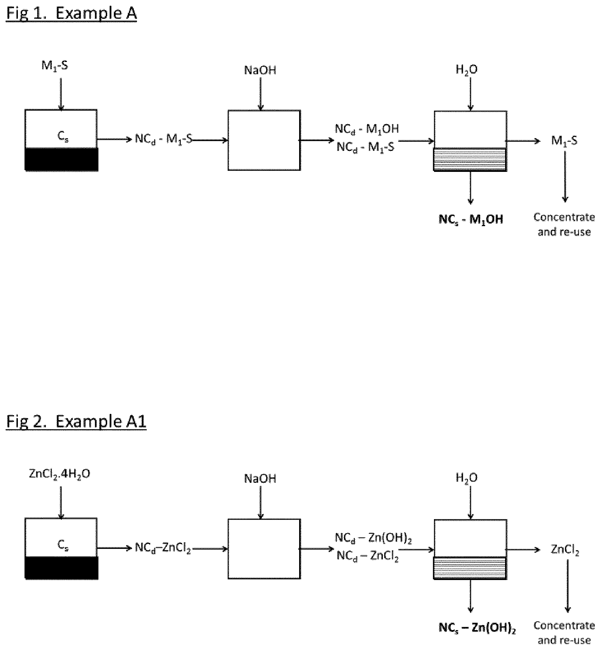

The following is a description of certain embodiments of the invention, given by way of example only and with reference to the drawings. Referring to Example A in FIG. 1, an embodiment of the process of the invention is shown wherein solid virgin cellulose (or virgin cellulose containing feed) $C_s$ is contacted with a molten metal salt solvent $M_1$-S ($M_1$ being the metal). FIG. 2 Example A1, shows a preferred embodiment wherein the molten metal salt solvent $M_1$-S is $ZnCl_2.4H_2O$ ($M_1$ is Zinc). In this first step the solid virgin cellulose $C_s$ is dissolved to form a suspension or solution of dissolved nano-cellulose in the molten metal salt ($NC_d$-$M_1$-S preferably $NC_d$-$ZnCl_2$). It is noted that the obtained product from the dissolution step comprises cellulose in nanocrystalline form and may also comprise cellulose polymer, oligomer or monomer that is molecularly dissolved, so the terms suspension and solution are used interchangeably for the product obtained in the dissolution process.

The cellulose particles obtained in this first step can be cellulose particles having XRD type I structure or XRD type II structure. However, it is preferred that the cellulose particles obtained are delaminated cellulose nano-particles having XRD type II structure before the metal precipitation reactant is added as this results in nano-cellulose with a higher aspect ratio and a hybrid composite wherein the cellulose - and metal compound nano-particles are more homogeneously mixed on nano- scale.

In a preferred embodiment, the cellulose particles obtained in a first step are cellulose particles having XRD type I structure which are converted in a separate second step to cellulose nano-particles having XRD type II structure. This second cellulose dissolution step is preferably before addition of the metal precipitation reactant as explained above.

Then a precipitation reactant is added to the obtained cellulose solution $NC_d$-$M_1$-S to convert at least part of the molten metal salt solvent $M_1$-S to an insoluble metal compound $M_1$-X that precipitates as nano-particles in the molten metal salt. In the embodiment shown in FIG. 1 and 2 the metal precipitation reactant is NaOH and the metal nano-particles formed are metal hydroxide $M_1$-OH nanoparticles. In the preferred embodiment of Example A1 in Fig 2. the obtained solution comprises dissolved nano cellulose $NC_d$ and $Zn(OH)_2$ nano-particles in the $ZnCl_2.4H_2O$ molten metal salt.

The addition of the metal precipitation reactant may also reduce the solvent quality of the molten salt solvent. It was observed that on addition of the precipitation reactant the cellulose nano-particles $NC_d$ start to form a cloudy gel with the precipitated metal compound precipitate $M_1$-OH ($Zn(OH)_2$ in Example A1) and only after adding anti-solvent a clear phase separation occurs between the $NC_d$- $M_1$-OH co-gel and the molten salt solvent.

In a next step an anti-solvent C ($H_2O$ in Example A and A1) is added reducing the solvent power of the molten metal salt and precipitating the cellulose nano-particles $NC_s$ together with the $M_1$-OH nano-particles (preferably . The precipitate is then separated to obtain the solid hybrid composite product $NC_s$-$Zn(OH)_2$. This separation step may involve filtering and/or centrifuging and may comprise one or more washing and/or drying steps. The process comprises steps a) - d) as successive steps without intermediate steps to reduce particle size. In particular, the process does not comprise, and does not need to comprise, milling, grinding, ultra-sonic treatment steps to reduce particle size.

Preferably the process also comprises a recycling step to regenerate the molten metal salt solvent $M_1$-S, which involves a step of concentrating the diluted molten salt for re-use in the first step. The anti-solvent preferably is water as it allows regeneration of the metal salt solvent $M_1$-S from the separated diluted solvent simply by evaporation of water to the concentration of the molten salt desired in the cellulose dissolution step.

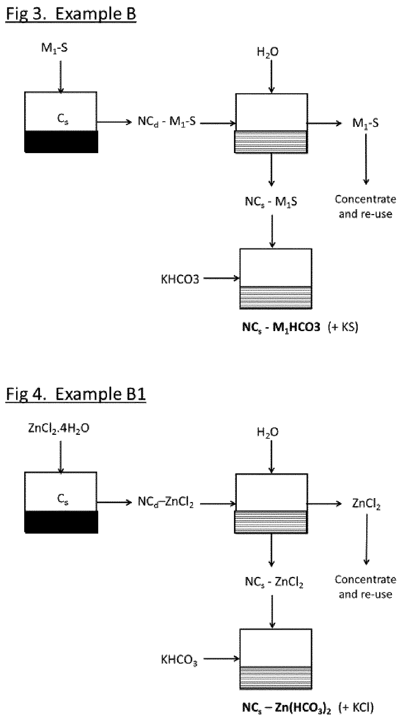

Examples A and A1 in Fig 1 and 2 illustrate the embodiment wherein the metal precipitation reactant is added before adding the anti-solvent to precipitate the nano-cellulose. Examples B and B1 in Fig 3 and 4, show an alternative embodiment of the process of the invention wherein the anti-solvent is added to precipitate the nano-cellulose before adding the metal precipitation reactant.

Herein first a solid virgin cellulose (containing feed) $C_S$ is contacted with a molten metal salt solvent

$M_1$-S to produce a solution of cellulose nano-particles, preferably of XRD type II, in the molten metal salt $NC_d$ - $M_1$-S in the same way as described above for Example A. Then anti-solvent C, preferably water, is added to produce a precipitate nano-cellulose $NC_s$ having a size typically below 200 nm to which metals $M_1$-S (preferably $ZnCl_2$) are attached in molecular form.

In a next step, a metal precipitation reactant is added to convert the molten metal salt solvent $M_1$-S to a metal compound $M_1$-X precipitating as nano-particles onto the precipitated nano-cellulose particles forming a very homogeneous hybrid composite of the metal compound and cellulose nanoparticles. In Example B and B1 the metal precipitation reactant is $KHCO_3$ and the hybrid composite comprises cellulose nano-particles $NC_s$ and nano-particles of the modified metal compound $M_1HCO_3$; preferably a hybrid composite $NC_s$ - $Zn(HCO_3)_2$ as shown in Example B1.

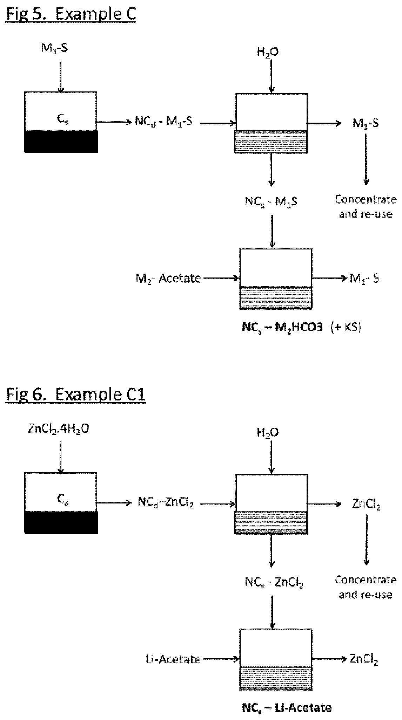

Examples C and C1 in FIG. 5 and 6 show a variant of Examples B and B1 wherein the metal precipitation reactant comprises a metal cation $M_2$ different from the metal $M_1$ in the molten metal solvent. In this embodiment the metal $M_1$ of the molten metal solvent is at least partially exchanged with metal cation $M_2$. Thereafter, the nano-particles of metal $M_2$ compound are formed on the precipitated cellulose nano-particles $NC_s$. In Example C the metal precipitation reactant is $M_2$-acetate and the hybrid composite formed comprises $M_2$-acetate and cellulose nano-particles $NC_s$. Example C1 shows a specific embodiment of the process using Li-acetate precipitation reactant to form a hybrid composite comprising Li-acetate and cellulose nanoparticles. This hybrid composite is useful for instance in solar cells. When the precipitation reactant water is added to the $ZnCl_2.nH_2O$ molten salt cellulose solution, the Cellulose precipitates with a lot of $ZnCl_2$ still present in the precipitate. Then the precipitate is washed with water to remove or reduce the amount of $ZnCl_2$ (what we normally do) or is washed with another metal salt $M_2X$ to replace $ZnCl_2$.

DETAILED DESCRIPTION OF THE INVENTION

The invention relates to a method for the preparation of a hybrid nano-structured composite comprising cellulose nano-particles and metal compound nano-particles, to the nano structured-composite product obtainable by the process and to uses thereof. The method comprises the steps of contacting virgin cellulose with a molten metal salt solvent $M_1$-S and dissoluting the virgin cellulose, optionally exchanging at least part of metal ions $M_1$ with metal ions $M_2$ and converting at least part of the metal ions $M_1$ and/or optional $M_2$ to metal compound nano-particles, precipitating the cellulose nano-particles and isolating the co-precipitated cellulose- and metal compound nano-particles.

The metal compound can be precipitated before or after precipitation of the cellulose nanoparticles. In a preferred embodiment the metal compound particles are precipitated after precipitation of the cellulose nano-particles. When the cellulose nano-particles are precipitated a sort of gel-like precipitate is formed in which the amount of metal ions $M_1$ of the molten salt metal compound is high. Preferably the amount of $M_1$ metal in the precipitate is reduced, preferably by filtering and washing, to an amount needed in view of the desired amount of metal compound nanoparticles on the cellulose nanoparticles followed by precipitating the metal compound nanoparticles $M_1$-X on the nanocellulose. If in view of the envisaged use of the hybrid composite a different metal $M_2$ is desired in the metal compound, a metal $M_2$ ion can be added to the molten salt and precipitating together with or instead of the metal $M_1$ compound. Preferably however, the metal ion $M_2$ is not present in the molten salt and the cellulose is precipitated first, followed by separating the molten $M_1$ metal salt solvent from the precipitated nano-cellulose for recycling and re-use in the first step and then contacting the precipitated nano-cellulose, which still is wetted with the molten metal $M_1$ salt solvent, with a metal $M_2$ salt in one or more steps to exchange the metal $M_1$ of the molten salt solvent with metal $M_2$ followed by precipitating the metal $M_2$ compound nanoparticles, optionally washing followed by drying.

The method according to the invention comprises as a first step a) the contacting virgin cellulose with a molten metal salt solvent $M_1$-S comprising metal ions $M_1$ and dissoluting the virgin cellulose. The molten metal salt M1-S preferably is a Zinc halogenide, more preferably Zinc-Chloride, Zinc-Bromide or hydrates thereof, and even more preferably $ZnCl_2.4H_2O$. The advantage of molten metal salt $ZnCl_2$ hydrate, preferably $ZnCl_2.4H_2O$ is that cellulose is well dissolved with little degradation and depolymerisation of the cellulose is reduced, in particular in the molten salt that is proton-free and preferably comprises a proton scavenger.

In one embodiment the molten metal $M_1$ salt solvent comprises a metal cation $M_2$ other than the metal $M_1$ of the molten salt solvent, preferably comprising a $ZnCl_2$ molten metal salt solvent and a $M_2$ metal chloride, wherein the metal cation $M_2$ preferably is one or more chosen from the group of Li, Mn, Ti, Zn, Nd, Cd, Ag and Ru, most preferably $TiCl_2$, $MnCl_2$, $LiCl_2$ and wherein preferably the amount of $M_2$ metal is less than 20%, preferably less than 10 or 5 mole % of the amount of $M_1$.

The cellulose XRD type II nano-particles nano-particles can be prepared in a process as described in WO2017/055407. However, a preferred process to prepare cellulose XRD type II nano-particles is described in WO2020212616. In this preferred embodiment the method of the invention comprises in step a) contacting virgin cellulose with a first solvent, characterized in that the first solvent is an aqueous solution comprising 40 - 65 wt.% $ZnCl_2$ in water, whereby in step b) the amorphous cellulose phase is preferentially dissolved over the crystalline cellulose phase, c) optionally separating the obtained crystalline cellulose having an XRD type I structure.

Cellulose having an XRD type II structure is then prepared in step d) comprising contacting the optionally separated crystalline cellulose having an XRD type I structure with a second solvent comprising a higher concentration than the first solvent of between 65 and 90 wt.% $ZnCl_2$ in water, wherein the second solvent and preferably also the first solvent is free of proton acid and preferably comprise a proton scavenger. In this process crystalline cellulose nano-particles having an XRD type II structure can be obtained in a high yield, high crystallinity and high purity (i.e. low amount of saccharide oligomers or -monomers and degradation products thereof). It is preferred that in this method the temperature in step a) and b) and preferably also of step d) is below 80°C, preferably below 70, 60 or even 50°C. It is possible to carry out the process at room temperature. Lower temperature presents milder conditions and increasing preference for dissolving only the amorphous phase but also increase the time needed to completion. Typically, higher concentration of $ZnCl_2$ is preferably combined with lower temperatures or visa-versa, lower concentration of $ZnCl_2$ can be combined with higher temperatures. Alternatively, it may be preferred that contacting step A is done at higher temperatures, for example between 50 and 80°C followed by quenching after a pre-determined optimum contacting time to prevent further dissolution of the crystalline cellulose. Quenching means quickly lowering the temperature and/ or quick dilution with water.

The method according to the invention comprises as step b) adding a precipitation reactant B to convert at least part of the metal ions $M_1$ of the molten metal salt solvent $M_1$-S to metal compound nano-particles $M_1$_X or exchanging at least part of metal ions $M_1$ with metal ions $M_2$ by contacting with a solution of a salt comprising metal ions $M_2$ and precipitating metal compound $M_2$_X nano-particles directly or by adding a precipitation reactant B.

The precipitation reactant B preferably is a base anion X, preferably comprising a hydroxy, carbonate or carboxylate, forming a nano-particle precipitate $M_1$-X with metal $M_1$ and/or $M_2$-X with metal $M_2$. The precipitation reactant B is preferably added as a salt comprising the base anion and hydrogen or a group IA metal cation, preferably sodium or potassium. The precipitation reactant B is chosen from the group of NaOH, KOH, $KHCO_3$, a formiate or acetate salt. The metal compound nanoparticles formed are for example nano-particles of $M_1$-OH, $M_1$-$HCO_3$, $M_1$-HCOO⁻ or $M_1$- CH;COO.

In another embodiment the precipitation reactant B comprises a metal ion $M_2$ other than metal $M_1$ or the metal $M_1$ is exchanged with metal $M_2$ to form a precipitate of metal $M_2$-X, wherein the metal cation $M_2$ is preferably one or more chosen from the group of Li, Mn, Ti, Nd, Cd, Ag and Ru.

The method according to the invention comprises as step c) adding an anti-solvent and precipitating the cellulose nano-particles before, during or after step b). Preferably, the antisolvent C is water, a ketone or alcohol, preferably water added in an amount to reduce the salt concentration of the molten salt, preferably the $ZnCl_2$ concentration, to between 10 and 30 wt.%, preferably between 15 and 25 wt.%. In step d) the obtained co-precipitated celluloseand metal compound nano-particles are isolated, preferably by filtration and/or centrifugation, optionally washed and dried to obtain the hybrid nano-structured composite material.

It is noted that WO2019229030A1 describes coprecipitation of cellulose with predispersed inorganic particles. This is different from the process of the invention wherein the metal of the molten metal salt is converted to form nano-particles on the nano-cellulose crystals which, as opposed to the prior art, achieves inorganic compound particles having significantly lower size of below 70, preferably below 50, more preferably below 30 and most preferably even below 20 nm. Another drawback of WO2019229030A1 is that it will only work with certain metal-oxides that allow to be dispersed in the molten salt solvent $ZnCl_2$. This is for instance not possible for oxides of 'heavier' metals like ZnO, NdO, Rare-Earth oxides etc.

In one embodiment of the method of the invention in step b) precipitation reactant B is added to convert at least part of the metal ions $M_1$ to a nano-particles of metal compound $M_1$. X followed by adding anti-solvent C in step c) to precipitate the cellulose nano-particles in the presence of the nano-particles of metal compound $M_1$.X, optionally followed by one or more steps of filtration, washing and drying.

In one embodiment of the method of the invention step a) is followed by step c) and then by step b) comprising adding anti-solvent C to the cellulose solution obtained in step a) forming a gel of precipitated cellulose nano-particles, optionally followed by separating the gel and washing to reduce the concentration of the metal salt of the molten salt solvent, followed by addition of precipitation reactant B in step b) to convert at least part of the metal ions $M_1$ to a nano-particles of metal compound $M_1$X in the presence of the cellulose nano-particles.

In a specific embodiment of this method, the molten metal salt solvent preferably is $ZnCl_2$.$4H_2O$ and the precipitation reactant B is a hydroxide base, preferably added as sodium hydroxide, resulting in a hybrid composite of cellulose- and Zinc-hydroxide nanoparticles. Optionally the obtained hybrid composite is then treated to convert Zinc-hydroxide nanoparticles to Zinc-oxide nano-particles, preferably by drying at elevated temperatures between 70 and 350°C, preferably between 80 and 300°C, even more preferably between 80 and 280°C.

In an alternative embodiment of the method of the invention step a) is followed by step c) wherein anti-solvent C is adding to the cellulose solution obtained in step a) forming a gel of precipitated cellulose nano-particles, optionally followed by separating the gel and washing to reduce the concentration of the metal salt of the molten salt solvent, followed by step b) wherein at least part of metal ions $M_1$ are exchanged with metal ions $M_2$ by contacting the solution with a solution of a salt comprising metal ions $M_2$, together with or followed addition of precipitation reactant B, forming precipitate of metal compound nano-particles $M_2\_X$.

The precipitation reactant B and the antisolvent C can be added separately as described above but can also be added together in a single combined step c) and d). Alternatively, a single compound is added that acts both as precipitation reactant B and as antisolvent C to simultaneously precipitate both the metal compound nano-particles and the cellulose nanoparticles. For example, the precipitation reactant B converts the metal $M_1$ of the molten salt into a precipitate and thereby influences the solubility of the dissolved cellulose to such extent that the cellulose precipitates. If precipitation reactant B is added as a solution of a salt, for example NaOH, in water, both the metal hydroxide compound and cellulose can precipitate.

The method according to the invention optionally comprises as step e) converting the metal compound in the hybrid nano-structured composite material into another metal compound. This can be done for example by thermal decomposition, ion-exchange, reduction or oxidation. Preferably, the metal compound in the hybrid nano-structured composite comprises a hydroxide, carbonate or carboxylate or combinations thereof, preferably carbonate hydroxide compounds, which are optionally converted to metal oxide by thermal decomposition, preferably under vacuum.

Optionally interlinking agents are added in the method to link with the cellulose nanoparticles, which are preferably chosen from the groups of glycerol, citric acid, acetate or chitosan and preferably are organometal compounds of exchange metal $M_2$, preferably $M_2$. acetate or -citrate and which interlinking agents are preferably added in step b) or c).

The invention also relates to hybrid nano-structured composite comprising cellulose nano-particles and metal compound nano-particles obtainable by the process according to the invention, preferably comprising 2-20 wt% metal compound relative to the total dry weight of the nano-structured composite. The hybrid nano-structured composite preferably comprises cellulose nano-particles having X-Ray Diffraction (XRD) type II structure that preferably have an aspect ratio AR of at least 5, preferably at least 10, preferably having a smallest size below 60, preferably below 40 and more preferably below 30 nm and comprising metal compound nano-particles having an average particle size below 80, preferably below 60 and more preferably below 40 nm and wherein the cellulose and metal compound nano-particles are homogeneously mixed on nano-scale. Preferred hybrid nano-structured composites of the invention comprise as metal compound Zinc-chloride, -hydroxide, -oxide or -carbonate, Lanthanum-chloride, -hydroxide, -oxide or -carbonate or lithium-acetate.

The invention also relates to the use of the hybrid nano-structured composite of the invention for energy generation, in electronic devices, as a pigment and/or a pigment support, as a whitener or filler in food or personal care products, as anti-bacterial compound, in antibacterial clothing, in the flexible and optically transparent paper, foil, tape or cloth.

The nano-cellulose based hybrid nanomaterials have huge potential applications in food packaging, biopharmaceuticals, biomedical, cosmetics and electronics. The hybrid composite combines the properties of functional metallic materials with nano-cellulose and can be used as a base material or building block in several applications. For example, the hybrid composites are used in certain electronic devices as for instance in solar cells, flexible displays, ultracapacitors etc. where metals like Li, Mn, Ti, Zn, Ru and others are combined with nano-cellulose paper, foils, sheets, tapes etc. The hybrid nanocomposite containing cellulose- and zinc-oxide nano-particles have excellent mechanical, UV barrier, and antibacterial properties.

In a particular aspect, the invention relates to the use of hybrid nano-structured composite wherein the metal compound is zinc-oxide or of cellulose nano-particles or of Zinc-oxide nano-particles or combinations thereof for replacing titanium-oxide as white pigment in food or personal care products or in paints, coatings and plastic articles.

The invention also relates to the use of the hybrid nano-structured composite of the invention in the catalytic conversion of cellulose into a performance chemical, preferably into an alcohol, a sugar or an acid, wherein the acid preferably is acetic or lactic acid. The metal compound in the hybrid nano-structured composite preferably is one or more chosen from the group of ZnO, BaO, PbO, SnO, FeO, CaO, MgO and $Al_2O_3$. More preferably the one or more metal compounds comprise ZnO. For example, a nano-structured hybrid composite comprising nanocellulose and 10 - 20 wt% of ZnO can be heated at a temperature between 150 and 300°C whereby the cellulose of the nanocellulose is converted into at least 25 wt% lactic acid relative to the weight of the nano-structured hybrid composite. An advantage of this method is that the ZnO nano-particle is very well distributed on nano-scale in the cellulose feedstock, which reduces mass transfer restrictions and time and lead to higher yields at higher selectivity.

**Experimental** Methods

Measurement of XRD crystal type

The cellulose products obtained in the experiments are characterised using XRD. XRD measurements according to the method described by: Z. Man, N. Muhammand, A. Sarwono, M.A. Bustam, M. Vignesh Kumar, S. Rafiq in J. Polym. Environ 19 (2011) 726-731: Preparation of cellulose nanocrystals using an Ionic liquid. The crystal type I or II was identified by peak positions, which are for type I on 2θ of 22.6° (the [200] reflection) and for type II on 2θ of 20° and 22° (the [110] and [020] reflection). Before XRD measurement the product samples were dried by vacuum drying at room temperature.

Measurement of XRD crystallinity

The product crystallinity (mentioned in the above document as crystallinity index) was determined using Segal's formula: $CrI = (I_{002}-I_{am})/I_{002}$ wherein $I_{002}$ is the overall intensity of the peak at 2θ of 22.6° for type I or 22° for type II cellulose and $I_{am}$ is the intensity of the baseline at 2θ about 18°.

Measurement of XRD crystal size

The cellulose crystal size was determined from the measured XRD using the Scherrer's equation:

$$\beta = \frac{0.9\lambda}{\tau \cos\theta}$$

wherein β is the crystallite sizes, λ is the wavelength of incident X-rays, τ is the full width at half maximum (FWHM) of the XRD peaks, θ is the diffraction angles corresponding to the planes.

Measurement of cellulose product yield and cellulose hydrolyzation

Soluble (poly-)sugars were measured based on mass balance % of (poly-)sugars = 1 - M $^{prec}_{cel}$/M $^{in}_{cel}$ wherein M $^{prec}_{cel}$ is the weight of dry micro- or nano-cellulose obtained in the experiment and M $^{in}_{cel}$ is the weight of dry cellulose placed in the reactor. The term (poly)sugars implies sugars and poly-sugars such as oligomer sugars. The drying of the obtained cellulose product is done according to the NREL lab procedure, convection oven drying for biomass is performed at 45° C for 24h - 48 h with regular (typically every 3 h) check of the weight until the dry biomass weight does not change more than 1 wt.% in one hour.

Measurement of aspect ratio and crystal size

The aspect ratio and crystal size of the nano-cellulose can be analysed with a scanning electron microscope (SEM) or transmission electron microscope.

Materials used

The cellulose base material in all the below described experiments is cotton linter Micro Crystalline Cellulose (MCC) ex-Sigma C6288. XRD characterization shows ± 80% of XRD-I type. $ZnCl_2$ and ZnO were also received from Sigma.

**DESCRIPTION OF EXPERIMENTS**

**Example 1: NC-II/Zn(OH)$_2$**

The first solvent was prepared by adding 0.5 g ZnO powder to 100 g aqueous solution of 60 wt.% $ZnCl_2$ in water, the mixture was kept under stirring (120 rpm/min) at room temperature overnight. Remaining unreacted ZnO solids were removed from the solution by filtration. The resulting 100 g solvent was mixed with 5 g of the cotton liner cellulose under stirring (480 rpm/min) and kept under stirring for 30 min at room temperature. The obtained cellulose type I crystals C-I were separated from the solution by filtration over a glass filter, washed 8 times with deionized water to remove $ZnCl_2$ and dried in vacuum at room temperature.
The highly crystalline cellulose type I crystals C-I were subsequently contacted with a second solvent. The second solvent is a molten metal salt solvent prepared by mixing 0.5 g ZnO powder (as proton scavenger) with 100 g aqueous solution of 70 to 75 wt% $ZnCl_2$ and kept under stirring at room temperature overnight. Remaining ZnO solids were removed from the solvent by filtration. 100 g of the second solvent was mixed with 5 g of the cellulose type I crystals C-I and stirred for 30 min at room temperature till the solution became clear.
225 g deionized water was added under stirring to the solution to decrease $ZnCl_2$ concentration to 20 wt.% to precipitate the cellulose from the second solution. The sample was kept under stirring for 20 min to allow the cellulose nanocrystals to precipitate. The precipitation causes the solution to gel. The gel precipitate is filtered. The cellulose to $ZnCl_2$ ratio in the gel precipitate is 1:10 based on dry solids weight.
The gel comprises high crystallinity high purity nano-cellulose having XRD type II (NC-II). XRD measurement of a dried sample of this precipitate shows that the cellulose XRD type I obtained in the first dissolution step is converted in the second step to nano cellulose XRD type II having an XRD crystallinity above 80% and less than 5 wt.% saccharide monomers or oligomers formed.
The gel precipitate is washed with water at T$_{room}$ = 25°C for 2 times with a gel-to-fresh water volume ratio 1:8 until the product has a $ZnCl_2$ content of about 10 wt% (cellulose to $ZnCl_2$ 10:1 based on dry solids). The resulting product is referred to as product 1-A.
An amount of 100 gr of this product 1-A is then mixed at temperature T = 25°C with 0.1 gr NaOH producing a cloudy gel-like solution which after filtration results in a wet precipitate comprising a mixture of precipitated nano-cellulose type II crystals and precipitated zinc-hydroxide NC-II/Zn(OH)$_2$. The amount of Zn(OH)$_2$ in the hybrid composite can be optimized by using an excess of NaOH to ensure complete Zn(OH)$_2$ precipitation, followed by washing out residual NaOH. The solid co-precipitate NC-II/Zn(OH)$_2$ is separated and washed twice with water (precipitate to water volume ratio 1:8) to remove NaCl. The precipitate sample is thereafter dried at 80 °C.

Example 2: NC-II/ZnO

In the above described preferred embodiment of Example 1 the nano-cellulose obtained have a high crystallinity and high purity and they degrade at higher temperature. It was found that high crystallinity high purity cellulose nanocrystals in dry state have excellent thermal stability and resist degradation at temperatures even above 200°C. This advantageous property can be used to thermally convert hybrid nano-structured composite of the invention at elevated temperatures. The precipitate sample NC-Zn(OH)$_2$ obtained in Example 1 is heated at 200°C to convert Zn(OH)$_2$ into ZnO. The final dry weight ratio cellulose to ZnO is 10: 0.75 as determined with DTA and XRF.

Example 3: NC-II/LaCl$_3$

An amount of 100 g of the NC-II/ZnCl$_2$ product 1-A is contacted with a 1000 ml solution of LaCl$_3$ (comprising 2 gr LaCl$_3$ in 1000 ml water) whereby the La exchanges the Zn forming a hybrid composition NC-II/LaCl$_3$ with relative amounts cellulose: LaCl$_3$: ZnCl$_2$ 10:1:0.1.

Example 4: NC-III La$_2$O$_3$

The product obtained in example B-1 is washed with NaOH to form La(OH)$_3$ which is converted by dehydration at T = 200°C under vacuum to La$_2$O$_3$ producing a NC-II/La$_2$O$_3$ hybrid composite.

Example 5: Whiteness Test of : NC-III La$_2$O$_3$

Nano-cellulose NC was produced separately as described in example 1 and instead of adding Na-OH metal precipitation reactant, the NC nano-crystals were washed, filtered and dried. ZnO nanoparticles were commercially obtained. The whiteness of the hybrid nano-structured composite material NC-II/ZnO of Example 2 was compared with the pure cellulose nanocrystals, ZnO nano-particles and white paper. It appeared that the NC-II/ZnO of Example 2 was whiter than the constituents as shown in the Table below.

| sample | whiteness value |
|---|---|
| White Paper | 88 |
| NC | 90 |
| ZnO | 89 |
| NC-ZnO | 92 |

Example 6: catalytic conversion of cellulose

A nano-structured hybrid composite comprising nanocellulose and 10 wt% of ZnO was heated to 150-300°C whereby the cellulose was converted into 25 wt% lactic acid relative to the weight of the nano-structured hybrid composite.

**Claims**

1. A method for preparing a hybrid nano-structured composite material comprising cellulose nanocrystalline particles and metal compound nano-particles comprising the steps of:

   a) Contacting virgin cellulose with a molten metal salt solvent M$_1$-S comprising metal ions M$_1$ and dissoluting the virgin cellulose,
   b) Adding a precipitation reactant B to convert at least part of the metal ions M$_1$ of the molten metal salt solvent M$_1$-S to metal compound nano-particles M$_1$X or exchanging at least part of metal ions M$_1$ with metal ions M$_2$ by contacting with a solution of a salt comprising metal ions M$_2$ and precipitating metal compound M$_2$X nano-particles directly or by adding a precipitation reactant B,
   c) Adding an anti-solvent and precipitating the cellulose nano-particles before, during or after step b),
   d) Isolating the co-precipitated cellulose- and metal compound nano-particles to obtain the hybrid nano-structured composite material,
   e) Optionally converting the metal compound in the hybrid nano-structured composite material into another metal compound.

2. The method of claim 1, wherein the molten metal salt M$_1$-S is a Zinc halogenide, preferably Zinc-Chloride, Zinc-Bromide or hydrates thereof, more preferably ZnCl$_2$ hydrate, most preferably ZnCl$_2$.4H$_2$O, which molten salt preferably is proton-free and preferably comprises a proton scavenger.

3. The method of claim 1 or 2, wherein the molten metal salt solvent M$_1$-S comprises a metal cation M$_2$ other than the metal M$_1$ of the molten salt solvent, wherein the metal cation M$_2$ preferably is one or more chosen from the group of Li, Mn, Ti, Zn, Nd, Cd, Ag and Ru, most preferably TiCl$_2$, MnCl$_2$ or LiCl$_2$ and wherein preferably the amount of M$_2$ metal is less than 20%, preferably less than 10 or 5 mole % of the amount of M$_1$ and wherein most preferably the molten metal salt solvent M$_1$-S is a ZnCl$_2$ hydrate according to claim 2 and the metal cation M$_2$ is in a M$_2$ metal chloride.

4. The method of anyone of claims 1-3, comprising in step a) contacting virgin cellulose with a first solvent, **characterized in that** the first solvent is an aqueous solution comprising 40 - 65 wt.% $ZnCl_2$ in water, whereby the amorphous cellulose phase is preferentially dissolved over the crystalline cellulose phase, optionally separating the obtained crystalline cellulose having an XRD type I structure, and contacting the optionally separated crystalline cellulose having an XRD type I structure with a second solvent comprising a higher concentration than the first solvent of between 65 and 90 wt.% $ZnCl_2$ in water to produce delaminated cellulose having an XRD type II structure, wherein the second solvent and preferably also the first solvent is free of proton acid and preferably comprise a proton scavenger.

5. The method of anyone of claims 1 - 4, wherein the precipitation reactant B is a base anion X, preferably comprising a hydroxy, carbonate or carboxylate, wherein reactant B is preferably chosen from the group of NaOH, KOH, $KHCO_3$, a formiate or acetate salt, wherein precipitation reactant B forms a nano-particle precipitate $M_1$-X with metal $M_1$ and/or $M_2$-X with metal $M_2$ and wherein optionally the precipitation reactant B may comprise a metal ion $M_2$ other than metal $M_1$ or wherein the metal $M_1$ is exchanged with metal $M_2$ to form a precipitate of metal $M_2$-X, wherein the metal cation $M_2$ is preferably one or more chosen from the group of Li, Mn, Ti, Nd, Cd, Ag and Ru.

6. The method of anyone of claims 1 - 5, wherein the anti-solvent C is water, a ketone or alcohol, preferably water added in an amount to reduce the salt concentration of the molten salt, preferably the $ZnCl_2$ concentration, to between 10 and 30 wt.%, preferably between 15 and 25 wt. %.

7. The method of anyone of claims 1 - 6, wherein in step b) precipitation reactant B is added to convert at least part of the metal ions $M_1$ to a nano-particles of metal compound $M_1$. X followed by adding anti-solvent C in step c) to precipitate the cellulose nano-particles in the presence of the nano-particles of metal compound $M_1$.X, optionally followed by one or more steps of filtration, washing and drying or preferably the method comprises step a) followed by step c) and then by step b) comprising adding anti-solvent C to the cellulose solution obtained in step a) forming a gel of precipitated cellulose nano-particles, optionally followed by separating the gel and washing to reduce the concentration of the metal salt of the molten salt solvent, followed by addition of precipitation reactant B in step b) to convert at least part of the metal ions $M_1$ to a nano-particles of metal compound $M_1$.X in the presence of the cellulose nano-particles.

8. The method of claim 7, wherein the molten metal salt solvent is $ZnCl_2.4H_2O$ and the precipitation reactant B is a hydroxide base resulting in a hybrid composite of cellulose- and Zinc-hydroxide nanoparticles and wherein optionally the hybrid composite is treated to convert Zinc-hydroxide nanoparticles to Zinc-oxide nano-particles, preferably by drying at elevated temperatures between 70 and 350°C, preferably between 80 and 300°C, even more preferably between 80 and 280°C.

9. The method of anyone of claims 1 - 8, wherein step a) is followed by step c) wherein anti-solvent C is adding to the cellulose solution obtained in step a) forming a gel of precipitated cellulose nano-particles, optionally followed by separating the gel and washing to reduce the concentration of the metal salt of the molten salt solvent, followed by step b) wherein at least part of metal ions $M_1$ are exchanged with metal ions $M_2$ by contacting the solution with a solution of a salt comprising metal ions $M_2$, together with or followed addition of precipitation reactant B, forming precipitate of metal compound nano-particles $M_2$.X.

10. The method of anyone of claims 1 - 9, comprising conversion step e) wherein the metal compound in the hybrid nano-structured composite material is converted into another metal compound, preferably by thermal decomposition, ion-exchange, reduction or oxidation.

11. The method of anyone of claims 1 - 10 wherein interlinking agents are added to link with the cellulose nanoparticles, which are preferably chosen from the group of glycerol, citric acid, acetate or chitosan and organometal compounds of exchange metal $M_2$, preferably organometal compounds of exchange metal $M_2$, more preferably $M_2$ -acetate or -citrate and which interlinking agents are preferably added in step b).

12. A hybrid nano-structured composite comprising cellulose nanocrystalline particles and metal compound nano-particles obtainable by the process of anyone of claims 1-11, preferably comprising 2-20 wt% metal compound relative to the total dry weight of the nano-structured composite, wherein the hybrid nano-structured composite preferably comprising cellulose nano-particles having X-Ray Diffraction (XRD) type II structure and preferably having an aspect ratio AR of at least 5, preferably at least 10, preferably having a smallest size below 60, preferably below 40 and more preferably below 30 nm and comprising metal compound nano-particles having an average particle size below 80, preferably below 60 and more preferably below 40 nm and wherein the cellulose and metal compound nano-particles are homogeneously mixed on nano-scale.

**13.** The hybrid nano-structured composite of claim 12 wherein the metal compound is Zinc-chloride, -hydroxide, -oxide or -carbonate, Lanthanum-chloride, -hydroxide, -oxide or - carbonate, lithium-acetate.

**14.** Use of the hybrid nano-structured composite of claims 12 or 13 for energy generation, in electronic devices, as a pigment and/or a pigment support, as a whitener or filler in food or personal care products, as anti-bacterial compound, in anti-bacterial clothing, in the flexible and optically transparent paper, foil, tape or cloth and preferably the use of the hybrid nano-structured composite comprising zinc-oxide as the metal compound, optionally in combination with cellulose nano-particles and/or Zinc-oxide nano-particles, for replacing titanium-oxide as white pigment in particular in food or personal care products, in paints, coatings or plastic articles.

**15.** Use of the hybrid nano-structured composite of claims 12 or 13 composite in the catalytic conversion of cellulose into a performance chemical, which performance chemical preferably is an alcohol, a sugar or an acid, more preferably acetic or lactic acid and wherein the metal compound in the hybrid nano-structured composite preferably is one or more chosen from the group of ZnO, BaO, PbO, SnO, FeO, CaO, MgO, $Al_2O_3$, more preferably the one or more metal compounds comprise ZnO.

**Patentansprüche**

**1.** Verfahren zum Herstellen eines nanostrukturierten Hybridwerkstoffmaterials, umfassend nanokristalline Zellulosepartikel und Metallverbindungsnanopartikel, umfassend die Schritte:

a) Inkontaktbringen von Frischzellulose mit einem geschmolzenen Metallsalzlösungsmittel Mi-S, umfassend Metallionen $M_1$ und Auflösen der Frischzellulose,
b) Zugeben eines Fällungsreaktanten B, um mindestens einen Teil der Metallionen $M_1$ des geschmolzenen Metallsalzlösungsmittels $M_1$-S zu Metallverbindungsnanopartikeln $M_1X$ umzuwandeln oder mindestens einen Teil der Metallionen $M_1$ mit Metallionen $M_2$ durch Inkontaktbringen mit einer Lösung eines Salzes, umfassend Metallionen $M_2$, auszutauschen und Metallverbindung $M_2X$-Nanopartikel direkt oder durch Zugeben eines Fällungsreaktanten B zu fällen,
c) Zugeben eines Antilösungsmittels und Fällen der Zellulosenanopartikel vor, während oder nach Schritt b),
d) Isolieren der gemeinsam gefällten Zellulose- und Metallverbindungsnanopartikel, um das nanostrukturierte Hybridwerkstoffmaterial zu erhalten,
e) Optionales Umwandeln der Metallverbindung in dem nanostrukturierten Hybridwerkstoffmaterial in eine andere Metallverbindung.

**2.** Verfahren nach Anspruch 1, wobei das geschmolzene Metallsalz $M_1$-S ein Zinkhalogenid, vorzugsweise Zinkchlorid, Zinkbromid oder Hydrate davon, mehr bevorzugt $ZnCl_2$-Hydrat, am meisten bevorzugt $ZnCl_2.4H_2O$, ist, wobei das geschmolzene Salz vorzugsweise protonenfrei ist und vorzugsweise einen Protonen-Scavenger umfasst.

**3.** Verfahren nach Anspruch 1 oder 2, wobei das geschmolzene Metallsalzlösungsmittel $M_1$-S ein Metallkation $M_2$ außer dem Metall $M_1$ des geschmolzenen Salzlösungsmittels umfasst, wobei das Metallkation $M_2$ vorzugsweise eines oder mehrere ist, die aus der Gruppe von Li, Mn, Ti, Zn, Nd, Cd, Ag und Ru, am meisten bevorzugt $TiCl_2$, $MnCl_2$ oder $LiCl_2$, ausgewählt sind, und wobei vorzugsweise die Menge an $M_2$ Metall weniger als 20 %, vorzugsweise weniger als 10 oder 5 Mol-% der Menge an $M_1$ beträgt und wobei am meisten bevorzugt das geschmolzene Metallsalzlösungsmittel $M_1$-S ein $ZnCl_2$-Hydrat nach Anspruch 2 ist und das Metallkation $M_2$ in einem $M_2$ Metallchlorid ist.

**4.** Verfahren nach einem der Ansprüche 1 bis 3, umfassend in Schritt a) das Inkontaktbringen von Frischzellulose mit einem ersten Lösungsmittel, **dadurch gekennzeichnet, dass** das erste Lösungsmittel eine wässrige Lösung ist, umfassend 40-65 Gew.-% $ZnCl_2$ in Wasser, wodurch die amorphe Zellulosephase gegenüber der kristallinen Zellulosephase bevorzugt gelöst wird, optional Trennen der erhaltenen kristallinen Zellulose, die eine XRD-Typ-I-Struktur aufweist, und Inkontaktbringen der optional getrennten kristallinen Zellulose, die eine XRD-Typ-I-Struktur aufweist, mit einem zweiten Lösungsmittel, umfassend eine höhere Konzentration als das erste Lösungsmittel von zwischen 65 und 90 Gew.-% $ZnCl_2$ in Wasser, um delaminierte Zellulose herzustellen, die eine XRD-Typ-II-Struktur aufweist, wobei das zweite Lösungsmittel und vorzugsweise ebenso das erste Lösungsmittel frei von Protonensäure sind und vorzugsweise einen Protonen-Scavenger umfassen.

**5.** Verfahren nach einem der Ansprüche 1 bis 4, wobei der Fällungsreaktant B ein Basenanion X ist, vorzugsweise umfassend ein Hydroxy, Karbonat oder Karboxylat, wobei Reaktant B vorzugsweise aus der Gruppe von NaOH, KOH, $KHCO_3$, einem Formiatoder Azetatsalz ausgewählt wird, wobei der Fällungsreaktant B eine Nanopartikelfällung $M_1X$ mit Metall $M_1$ und/oder $M_2X$ mit Metall $M_2$ ausbildet und

wobei optional der Fällungsreaktant B ein Metallion $M_2$ außer Metall $M_1$ umfassen kann oder wobei das Metall $M_1$ mit Metall $M_2$ ausgetauscht wird, um eine Fällung des Metalls $M_2\_X$ auszubilden, wobei das Metallkation $M_2$ vorzugsweise eines oder mehrere ist, die aus der Gruppe von Li, Mn, Ti, Nd, Cd, Ag und Ru ausgewählt sind.

6. Verfahren nach einem der Ansprüche 1 bis 5, wobei das Antilösungsmittel C Wasser, ein Keton oder Alkohol ist, vorzugsweise Wasser, das in einer Menge zugegeben wird, um die Salzkonzentration des geschmolzenen Salzes, vorzugsweise der $ZnCl_2$-Konzentration, auf zwischen 10 und 30 Gew.%, vorzugsweise zwischen 15 und 25 Gew.%, zu verringern.

7. Verfahren nach einem der Ansprüche 1 bis 6, wobei in Schritt b) der Fällungsreaktant B zugegeben wird, um mindestens einen Teil der Metallionen $M_1$ zu Nanopartikeln der Metallverbindung $M_1\_X$ umzuwandeln, gefolgt von dem Zugeben des Antilösungsmittels C in Schritt c), um die Zellulosenanopartikel in der Gegenwart der Nanopartikel der Metallverbindung $M_1\_X$ zu fällen, optional gefolgt von einem oder mehreren Schritten einer Filtration, eines Waschens und eines Trocknens, oder vorzugsweise das Verfahren Schritt a) umfasst, gefolgt von Schritt c) und dann Schritt b), umfassend das Zugeben des Antilösungsmittels C zu der Zelluloselösung, die in Schritt a) erhalten wird, wodurch ein Gel aus gefällten Zellulosenanopartikeln ausgebildet wird, optional gefolgt von dem Trennen des Gels und dem Waschen, um die Konzentration des Metallsalzes des geschmolzenen Salzlösungsmittels zu verringern, gefolgt von einer Zugabe des Fällungsreaktanten B in Schritt b), um mindestens einen Teil der Metallionen $M_1$ zu Nanopartikeln der Metallverbindung $M_1\_X$ in der Gegenwart der Zellulosenanopartikel umzuwandeln.

8. Verfahren nach Anspruch 7, wobei das Lösungsmittel des geschmolzenen Metallsalzes $ZnCl_2.4H_2O$ ist und der Fällungsreaktant B eine Hydroxidbase ist, was zu einem Hybridwerkstoff aus Zellulose- und Zinkhydroxidnanopartikeln führt, und wobei optional der Hybridwerkstoff behandelt wird, um Zinkhydroxidnanopartikel in Zinkoxidnanopartikel umzuwandeln, vorzugsweise durch Trocknen bei erhöhten Temperaturen zwischen 70 und 350 °C, vorzugsweise zwischen 80 und 300 °C, noch mehr bevorzugt zwischen 80 und 280 °C.

9. Verfahren nach einem der Ansprüche 1 bis 8, wobei Schritt a) von Schritt c) gefolgt ist, in dem das Antilösungsmittel C zu der Zelluloselösung gibt, die in Schritt a) erhalten wird, wodurch ein Gel aus gefällten Zellulosenanopartikeln ausgebildet wird, optional gefolgt von dem Trennen des Gels und dem Waschen, um die Konzentration des Metallsalzes des geschmolzenen Salzlösungsmittels zu verringern, gefolgt von Schritt b), in dem mindestens ein Teil der Metallionen $M_1$ durch Metallionen $M_2$ durch Inkontaktbringen der Lösung mit einer Lösung eines Salzes ausgetauscht werden, umfassend Metallionen $M_2$, zusammen mit oder gefolgt von der Zugabe des Fällungsreaktanten B, wodurch eine Fällung aus Metallverbindungsnanopartikeln $M_2\_X$ ausgebildet wird.

10. Verfahren nach einem der Ansprüche 1 bis 9, umfassend den Umwandlungsschritt e), bei dem die Metallverbindung in dem nanostrukturierten Hybridwerkstoffmaterial in eine andere Metallverbindung umgewandelt wird, vorzugsweise durch thermische Zersetzung, Ionenaustausch, Reduktion oder Oxidation.

11. Verfahren nach einem der Ansprüche 1 bis 10, wobei Vernetzungsmittel zugegeben werden, um sich mit den Zellulosenanopartikeln zu vernetzen, die vorzugsweise aus der Gruppe von Glycerin, Zitronensäure, Azetat oder Chitosan und Organometallverbindungen des Austauschmetalls $M_2$, vorzugsweise Organometallverbindungen des Austauschmetalls $M_2$, mehr bevorzugt $M_2$-Azetat oder -zitrat ausgewählt werden, und welche Vernetzungsmittel bevorzugt in Schritt b) zugegeben werden.

12. Nanostrukturierter Hybridwerkstoff, umfassend nanokristalline Zellulosepartikel und Metallverbindungsnanopartikel, die durch den Prozess nach einem der Ansprüche 1 bis 11 erhältlich sind, und vorzugsweise umfassend zu 2 bis 20 Gew.-% Metallverbindung relativ zu dem Gesamttrockengewicht des nanostrukturierten Werkstoffs, wobei der nanostrukturierte Hybridwerkstoff vorzugsweise Zellulosenanopartikel umfasst, die eine Struktur vom Typ II gemäß Röntgenbeugung (XRD) aufweisen und vorzugsweise ein Aspektverhältnis AR von mindestens 5, vorzugsweise mindestens 10, aufweisen, vorzugsweise eine kleinste Größe von unter 60, vorzugsweise unter 40 und mehr bevorzugt unter 30 nm aufweisen und umfassend Metallverbindungsnanopartikel, die eine durchschnittlichen Partikelgröße von unter 80, vorzugsweise unter 60 und mehr bevorzugt unter 40 nm aufweisen und wobei die Zellulose- und Metallverbindungsnanopartikel in dem Nanomaßstab homogen gemischt sind.

13. Nanostrukturierter Hybridwerkstoff nach Anspruch 12, wobei die Metallverbindung Zinkchlorid, -hydroxid, -oxid oder -karbonat, Lanthanchlorid, -hydroxid, -oxid oder -karbonat oder Lithiumazetat ist.

14. Verwendung des nanostrukturierten Hybridwerkstoffs der Ansprüche 12 oder 13 für eine Energieerzeugung, in elektronischen Vorrichtungen, als ein Pigment und/oder ein Pigmentträger, als ein Weißmacher oder ein Füllstoff in Lebensmitteln oder Körperpflegeprodukten, als antibakterielle Verbindung, in antibakterieller Kleidung, in dem flexiblen und optisch transparenten Papier, Folie, Band oder Stoff und vorzugsweise die Verwendung des nanostrukturierten Hybridwerkstoffs umfassend Zinkoxid als die Metallverbindung, optional in Kombination mit Zellulosenanopartikeln und/oder Zinkoxidnanopartikeln, zum Ersetzen von Titanoxid als Weißpigment insbesondere in Lebensmitteln oder Körperpflegeprodukten, in Farben, Beschichtungen oder Kunststofferzeugnissen.

15. Verwendung des nanostrukturierten Hybridwerkstoffs nach Anspruch 12 oder 13 bei der katalytischen Umwandlung von Zellulose in eine Leistungschemikalie, wobei die Leistungschemikalie vorzugsweise ein Alkohol, ein Zucker oder eine Säure, mehr bevorzugt Essig- oder Milchsäure ist, und wobei die Metallverbindung in dem nanostrukturierten Hybridwerkstoff vorzugsweise eines oder mehrere ist, die aus der Gruppe von ZnO, BaO, PbO, SnO, FeO, CaO, MgO, $Al_2O_3$ ausgewählt ist, mehr bevorzugt wobei die eine oder die mehreren Metallverbindungen ZnO umfassen.

**Revendications**

1. Procédé destiné à préparer un matériau composite hybride nano-structuré comprenant des particules nanocristallines de cellulose et des nanoparticules de composé métallique, comprenant les étapes consistant à :

   a) La mise en contact de la cellulose vierge avec un solvant de sel métallique fondu $M_1$-S comprenant des ions métalliques $M_1$ et la dissolution de la cellulose vierge,
   b) L'ajout d'un réactif de précipitation B pour convertir au moins une partie des ions métalliques $M_1$ du solvant de sel métallique fondu $M_1$-S en nanoparticules de composé métallique $M_1.X$ ou l'échange d'au moins une partie des ions métalliques $M_1$ avec des ions métalliques $M_2$ par contact avec une solution d'un sel comprenant des ions métalliques $M_2$ et la précipitation des nanoparticules de composé métallique $M_2.X$ directement ou par l'ajout d'un réactif de précipitation B,
   c) L'ajout d'un anti-solvant et la précipitation des nanoparticules de cellulose avant, pendant ou après l'étape b),
   d) L'isolement des nanoparticules de cellulose et de composés métalliques coprécipités pour obtenir le matériau composite hybride nano-structuré,
   e) La conversion, éventuellement, du composé métallique du matériau composite nano-structuré hybride en un autre composé métallique.

2. Procédé selon la revendication 1, dans lequel le sel métallique fondu $M_1$-S est un halogénure de zinc, de préférence, un chlorure de zinc, un bromure de zinc ou des hydrates de ceux-ci, plus préférablement, un hydrate de $ZnCl_2$, le plus préférablement, $ZnCl_2.4H_2O$, lequel sel fondu est de préférence exempt de protons et comprend, de préférence, un piégeur de protons.

3. Procédé selon la revendication 1 ou 2, dans lequel le solvant de sel métallique fondu $M_1$-S comprend un cation métallique $M_2$, autre que le métal $M_1$ du solvant de sel fondu, dans lequel le cation métallique $M_2$ est, de préférence, un ou plusieurs choisis dans le groupe de Li, Mn, Ti, Zn, Nd, Cd, Ag et Ru, le plus préférablement, $TiCl_2$, $MnCl_2$ ou $LiCl_2$ et dans lequel, de préférence, la quantité de métal $M_2$ est inférieure à 20 %, de préférence, inférieure à 10 ou 5 % molaire de la quantité de $M_1$ et dans lequel, le plus préférablement, le solvant de sel métallique fondu $M_1$-S est un hydrate de $ZnCl_2$ selon la revendication 2 et le cation métallique $M_2$ est dans un chlorure métallique $M_2$.

4. Procédé selon l'une quelconque des revendications 1 à 3, comprenant, dans l'étape a), la mise en contact de la cellulose vierge avec un premier solvant, **caractérisé en ce que** le premier solvant est une solution aqueuse comprenant 40 à 65 % en poids de ZnCl2 dans de l'eau, selon lequel la phase de cellulose amorphe est préférablement dissoute par rapport à la phase de cellulose cristalline, en séparant, éventuellement, la cellulose cristalline obtenue ayant une structure XRD de type I, et en mettant en contact la cellulose cristalline, éventuellement, séparée ayant une structure XRD de type I avec un second solvant comprenant une concentration plus élevée que le premier solvant, comprise entre 65 et 90 % en poids de $ZnCl_2$ dans l'eau pour produire de la cellulose délaminée ayant une structure XRD de type II, dans lequel le second solvant et, de préférence, aussi, le premier solvant, sont exempts d'acide protonique et comprennent, de préférence, un piégeur de protons.

5. Procédé selon l'une quelconque des revendications 1 à 4, dans lequel le réactif de précipitation B est un anion de base X, comprenant, de préférence, un hydroxy, un carbonate ou un carboxylate, dans lequel le réactif B est, de préférence, choisi dans le groupe de NaOH, KOH, $KHCO_3$, un formiate ou un sel d'acétate, dans lequel le réactif de précipitation B forme un précipité de nanoparticules $M_1$-X avec le métal $M_1$ et/ou $M_2$.X avec le métal $M_2$ et dans lequel, éventuellement, le réactif de précipitation B peut comprendre un ion métallique $M_2$ autre que le métal $M_1$ ou dans lequel le métal $M_1$ est échangé avec le métal $M_2$ pour former un précipité de métal $M_2$-X, dans lequel le cation métallique $M_2$ est de préférence un ou plusieurs choisis dans le groupe de Li, Mn, Ti, Nd, Cd, Ag et Ru.

6. Procédé selon l'une quelconque des revendications 1 à 5, dans lequel l'anti-solvant C est de l'eau, une cétone ou un alcool, de préférence, de l'eau ajoutée en quantité suffisante pour réduire la concentration de sel du sel fondu, de préférence, la concentration de $ZnCl_2$ est comprise entre 10 et 30 % en poids, de préférence, entre 15 et 25 % en poids.

7. Procédé selon l'une quelconque des revendications 1 à 6, dans lequel, dans l'étape b), le réactif de précipitation B est ajouté pour convertir au moins une partie des ions métalliques $M_1$ en nanoparticules de composé métallique $M_1$. X, suivi de l'ajout de l'anti-solvant C, dans l'étape c), pour précipiter les nanoparticules de cellulose en présence des nanoparticules du composé métallique $M_1$.X, éventuellement, suivie d'une ou plusieurs étapes de filtration, de lavage et de séchage ou, de préférence, le procédé comprend l'étape a) suivie de l'étape c), puis de l'étape b) comprenant l'ajout de l'anti-solvant C à la solution de cellulose obtenue à l'étape a) pour former un gel de nanoparticules de cellulose précipitées, éventuellement, suivie d'une séparation du gel et d'un lavage pour réduire la concentration du sel métallique du solvant de sel fondu, suivie de l'ajout du réactif de précipitation B, dans l'étape b), pour convertir au moins une partie des ions métalliques $M_1$ en nanoparticules de composé métallique $M_1$.X en présence des nanoparticules de cellulose.

8. Procédé selon la revendication 7, dans lequel le solvant de sel métallique fondu est $ZnCl_2.4H_2O$ et le réactif de précipitation B est une base d'hydroxyde résultant en un composite hybride de nanoparticules de cellulose et d'hydroxyde de zinc et dans lequel le composite hybride est éventuellement traité pour convertir les nanoparticules d'hydroxyde de zinc en nanoparticules d'oxyde de zinc, de préférence, par séchage à des températures élevées comprises entre 70 et 350 °C, de préférence, entre 80 et 300 °C, encore plus préférablement entre 80 et 280 °C.

9. Procédé selon l'une quelconque des revendications 1 à 8, dans lequel l'étape a) est suivie d'une étape c), dans lequel l'anti-solvant C est ajouté à la solution de cellulose obtenue à l'étape a), formant un gel de nanoparticules de cellulose précipitées, éventuellement, suivi d'une séparation du gel et d'un lavage pour réduire la concentration du sel métallique du solvant de sel fondu, suivi de l'étape b), dans lequel au moins une partie des ions métalliques $M_1$ sont échangés avec des ions métalliques $M_2$ par contact de la solution avec une solution d'un sel comprenant des ions métalliques $M_2$, avec ou après l'ajout du réactif de précipitation B, formant un précipité de nanoparticules de composé métallique $M_2$.X.

10. Procédé selon l'une quelconque des revendications 1 à 9, comprenant l'étape de conversion e), dans lequel le composé métallique du matériau composite nano-structuré hybride est converti en un autre composé métallique, de préférence, par décomposition thermique, échange d'ions, réduction ou oxydation.

11. Procédé selon l'une quelconque des revendications 1 à 10, dans lequel des agents d'interliaison sont ajoutés pour se lier aux nanoparticules de cellulose, qui sont, de préférence, choisis dans le groupe du glycérol, de l'acide citrique, de l'acétate ou du chitosane et des composés organométalliques du métal d'échange $M_2$, de préférence, des composés organométalliques du métal d'échange $M_2$, plus préférablement, de l'acétate ou du citrate $M_2$, et lesquels agents d'interliaison sont, de préférence, ajoutés lors de l'étape b).

12. Composite nano-structuré hybride comprenant des particules nanocristallines de cellulose et des nanoparticules de composé métallique obtenues par le procédé selon l'une quelconque des revendications 1 à 11, comprenant, de préférence, de 2 à 20 % en poids de composé métallique par rapport au poids sec total du composite nano-structuré, dans lequel le composite nano-structuré hybride comprend, de préférence, des nanoparticules de cellulose ayant une structure de type II de diffractométrie de rayons X (DRX) et ayant, de préférence, un rapport d'aspect AR d'au moins 5, de préférence, au moins 10, ayant, de préférence, une plus petite taille inférieure à 60, de préférence, inférieure à 40 et de préférence inférieure à 30 nm et comprenant des nanoparticules de composés métalliques ayant une taille moyenne inférieure à 80, de préférence, inférieure à 60 et de préférence, inférieure à 40 nm et dans lequel les nanoparticules de cellulose et de composés métalliques sont mélangées de manière homogène à l'échelle nanométrique.

13. Composite hybride nano-structuré selon la revendication 12, dans lequel le composé métallique est du chlorure, hydroxyde, oxyde ou carbonate de zinc, du chlorure, hydroxyde, oxyde ou carbonate de lanthane, de l'acétate de lithium.

14. Utilisation du composite hybride nano-structuré selon l'une quelconque des revendications 12 ou 13, pour la génération d'énergie, dans des dispositifs électroniques, comme pigment et/ou support de pigment, comme blanchisseur ou charge dans des produits alimentaires ou de soins personnels, comme composé antibactérien, dans des vêtements antibactériens, dans le papier, feuille, ruban ou tissu flexibles et optiquement transparents, de préférence, l'utilisation du composite hybride nano-structuré comprenant de l'oxyde de zinc comme composé métallique, éventuellement, en combinaison avec des nanoparticules de cellulose et/ou des nanoparticules d'oxyde de zinc, pour remplacer l'oxyde de titane comme pigment blanc, en particulier dans les produits alimentaires ou de soins personnels, dans les peintures, revêtements ou articles en plastique.

15. Utilisation du composite hybride nano-structuré selon l'une quelconque des revendications 12 ou 13, dans la conversion catalytique de la cellulose en un produit chimique de performance, lequel produit chimique de performance est, de préférence, un alcool, un sucre ou un acide, plus préférablement l'acide acétique ou lactique et, dans lequel le composé métallique dans le composite hybride nano-structuré est de préférence un ou plusieurs choisis dans le groupe de ZnO, BaO, PbO, SnO, FeO, CaO, MgO, $Al_2O_3$, plus préférablement l'un ou plusieurs composés métalliques comprennent ZnO.

Fig 1. Example A

Fig 2. Example A1

## Fig 3.  Example B

M$_1$-S → C$_s$ → NC$_d$ - M$_1$-S → H$_2$O → M$_1$-S

NC$_s$ - M$_1$S

Concentrate and re-use

KHCO3 →

**NC$_s$ - M$_1$HCO3**  (+ KS)

## Fig 4.  Example B1

ZnCl$_2$.4H$_2$O → C$_s$ → NC$_d$–ZnCl$_2$ → H$_2$O → ZnCl$_2$

NC$_s$ - ZnCl$_2$

Concentrate and re-use

KHCO$_3$ →

**NC$_s$ – Zn(HCO$_3$)$_2$**  (+ KCl)

## Fig 5.  Example C

## Fig 6.  Example C1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017055407 A
- WO 2020212616 A
- WO 2019229030 A1

**Non-patent literature cited in the description**

- **DELGADO-AGUILAR et al.** *BioResources*, 2015, vol. 10 (3), 5345-5355
- *International Journal of Biological Macromolecules*, 2020, vol. 154, 1050-1073
- **MA et al.** *Cellulose*, 2016, vol. 23, 3703-3715
- **RUSZALA et al.** *IJCEA*, October 2015, vol. 6 (5), 331-340
- **Z. MAN ; N. MUHAMMAND ; A. SARWONO ; M.A. BUSTAM ; M. VIGNESH KUMAR ; S. RAFIQ.** *J. Polym. Environ*, 2011, vol. 19, 726-731